# EUROPEAN PATENT APPLICATION

(11) **EP 3 627 155 A1**
(43) Date of publication of application: **25.03.2020**
(21) Application number: 18802341.0
(22) Date of filing: 17.05.2018
(51) Int. Cl.: G01N 33/574

(54) **PEPTIDES DERIVED FROM TRUNCATED SOMATOSTATIN RECEPTOR SST5TMD4 AS BIOMARKERS AND THERAPEUTIC TARGETS IN TUMOURS**

(30) Priority: 17.05.2017 ES 201730702
(71) Applicant: Universidad De Córdoba, 14071 Córdoba (ES); Servicio Andaluz de Salud, 41410 Sevilla (ES)
(72) Inventor: CASTA O FUENTES, Justo P., 14004 C rdoba (ES); DEL RIO MORENO, Mercedes, 14004 C rdoba (ES); ALORS PEREZ, Emilia, 14004 C rdoba (ES); GAHETE ORTIZ, Manuel D., 14004 Cordoba (ES); LUQUE HUERTAS, Ra l, 14004 Cordoba (ES)
(74) Representative: Hoffmann Eitle
(86) International application number: PCT/ES2018/070361
(87) International publication number: WO 2018/211162

(57) **Abstract**

The present invention relates to the use of peptides derived from the extracellular end of truncated receptor sst5TMD4 as markers in a method for the diagnosis and/or prognosis of cancer in a patient, and to a diagnostic kit or device. The present invention also relates to the use of peptides derived from the extracellular end of truncated receptor sst5TMD4 as therapeutic targets in cancer.

## Description

### Technical Field

The present invention is comprised in the field of the biomedicine, and in particular may provide a method for non-invasive diagnosis of cancer which comprises quantifying levels of peptides derived from truncated somatostatin receptor sst5TMD4 in plasma, serum, blood, and/or urine of patients. Similarly, the present invention may also provide a new therapeutic target which involves blocking the action that these peptides exert on the tumor cells to increase their aggressiveness.

### Prior Art

Tumors constitute one of the most serious and complex world health threats of the 21^{st} century (Siegel, Miller *et al.* 2017). In fact, more than 200,000 people die every year in Spain alone as the result of a type of cancer (source: AECC (Spanish Association against Cancer)). For this reason, some of the most outstanding current lines of research in biomedical sciences seek to fight against conditions of this type through the identification of new molecular markers which aid in the suitable diagnosis and/or prognosis of a tumor, as well as new cellular and molecular mechanisms and elements which serve to identify and develop more effective therapeutic targets.

In this sense, a neuropeptide fundamentally produced in the central nervous system and in the gastrointestinal tract which exerts a wide range of biological functions, both in endocrine and non-endocrine tissues, is of special interest in the somatostatin system, among which the inhibition of hormone secretion and cell growth stand out (Patel 1999, Viollet, Lepousez *et al.* 2008). For this reason, synthetic analogues of somatostatin are currently used for the medical treatment of certain types of tumors with an endocrine component (Luque, Ibanez-Costa *et al.* 2015). Namely, somatostatin or its analogs perform their functions through the activation of 7 transmembrane domains of a family of G protein-coupled receptors (GPCRs), referred to as somatostatin or ssts receptors. This family is made up of five receptors (sst1-5) encoded in different genes (Csaba, Peineau *et al.* 2012) which have been widely characterized and classified. In fact, it seems that subtypes sst2 and sst5 are involved in the control of hormone secretion as well as in cell growth, whereas sst1, sst3, and sst4 are primarily involved in non-endocrine functions (Pisarek, Stepien *et al.* 2009, Klagge, Krause *et al.* 2010, Unger, Ueberberg *et al.* 2012). However, despite the fact that these receptors are present in samples and cells from different tumors (breast, prostate, liver, pituitary gland, or neuroendocrine tumors), the application of somatostatin analogs in the treatment of pathologies of this type has shown partial success in only some of these pathologies (Watt, Kharmate *et al.* 2008).

Recent studies have shown the existence of various mechanisms which increase the variability of GPCRs, including alternative splicing processes which would generate the occurrence of splicing variants with less than 7 transmembrane domains (McWilliams, Watson *et al.* 1998, Kilpatrick, Dautzenberg *et al.* 1999, Rekasi, Czompoly *et al.* 2000, Hawrylyshyn, Michelotti *et al.* 2004, Havt, Schally *et al.* 2005). These truncated receptors can possess characteristic functions and/or regulate those functions of their respective canonical isoforms (Kilpatrick, Dautzenberg *et al.* 1999). Furthermore, these variants are frequently associated with tumors, such as the case of the GH-releasing hormone receptor (Rekasi, Czompoly *et al.* 2000, Havt, Schally *et al.* 2005), cholecystokinin receptor (McWilliams, Watson *et al.* 1998), or adrenoreceptors (Hawrylyshyn, Michelotti *et al.* 2004). In this same line, the present group has recently identified two new variants of human subtype sst5, generated by non-canonical, alternative splicing exhibiting unique molecular and functional characteristics and called sst5TMD5 (with 5 transmembrane domains) and sst5TMD4 (with 4 transmembrane domains). Prior studies have demonstrated that truncated somatostatin receptor sst5TMD4 is overexpressed in some cancers such as breast cancer (Duran-Prado, Gahete *et al.* 2012), prostate cancer, and thyroid cancer (Puig-Domingo, Luque *et al.* 2014), as well as in pituitary gland tumors (Luque, Ibanez-Costa *et al.* 2015) and neuroendocrine tumors (Sampedro-Nunez, Luque *et al.* 2015), where it seems to play a relevant role in tumor malignancy and could be related to the lack of a response to treatment with somatostatin analogs (Duran-Prado, Saveanu *et al.* 2010). However, the mechanisms of action and underlying molecular basis with respect to such associations have not been completely explained.

In this sense, it should be pointed out that this receptor possesses only 4 transmembrane domains, as opposed to the 7 domains that classical receptors have, therefore its C-terminal tail is exposed outside the cell rather than towards the cytoplasm. This particular characteristic may suggest that, as occurs in the case of the GH receptor (GHR) (Havt, Schally *et al.* 2005), this end is susceptible to the action of proteases located in the extracellular matrix, such as matrix metalloproteinases (MMPs) or in the plasma membrane, such that one or more peptides derived from the extracellular domain of receptor sst5TMD4 which could exert a pathophysiological function as they are cleaved are generated.

Namely, matrix metalloproteinases or MMPs are a family of zinc- and calcium-dependent proteolytic enzymes which can be anchored to the membrane or be secreted (Brinckerhoff and Matrisian 2002). In human beings, a total of 28 isoforms have been described, some of which are involved in the development and/or progression of different tumors (Davies 2014). These enzymes are made up of different domains and motifs which are the basis for their classification and are important for their substrate specificity, location, and interactions with other macromolecules. Together, MMPs can degrade most proteins in the extracellular matrix, as well as regulate the activity of other proteinases, growth factors, cytokines, chemokines, and receptors of the cells (Butler and Overall 2009). By means of the excision of proteins of the extracellular matrix as well as other molecules, MMPs can modify signaling routes, therefore being central regulators of cell function under both physiological and pathological conditions (Nagase, Visse *et al*. 2006, Butler and Overall 2009, Hadler-Olsen, Fadnes *et al.* 2011). In fact, the expression of many members of the family of MMPs is induced both in cancer cells and in the surrounding stroma. The main mechanism whereby they promote the spread of cancer is by degradation of the extracellular matrix. This family of enzymes can thereby affect many of the central processes involved in tumorigenesis and progression of cancer, such as growth, survival, angiogenesis, invasion, and regulation of the immune response, therefore being important modulators in tumor progression (Hadler-Olsen, Winberg *et al.* 2013). It is currently known that there are high levels of MMP-1, 2, 3, 7, 8, 9, 11, 13, 14, 15, and 26 in blood, urine, and/or cancerous tissue from patients with various pathologies (Jiang, Davies *et al.* 2006, Somiari, Somiari *et al.* 2006, Kohrmann, Kammerer *et al.* 2009, Zhao, Xiao *et al.* 2009, Cheng, Yu *et al.* 2010, Stankovic, Konjevic *et al.* 2010), with this overexpression being related to the *in vitro* invasiveness of cancer cells (Balduyck, Zerimech *et al.* 2000, Chen and Thompson 2003, Magee, McGlynn *et al.* 2004).

In fact, the extracellular C-terminal end of truncated receptor sst5TMD4 presents cleavage sites for certain MMPs, such that the action of these enzymes could generate the release of peptides with pro-tumorigenic actions. Namely, an *in silico* approach has determined two possible cleavage sites for metalloproteinases MMP-2, -9, -14, and/or MMP-16, the processing of which could generate three soluble fragments with 7, 10, or 17 amino acids (M7, M10, and M17, respectively) (Figure 1) which could be released into bodily fluids (blood and/or urine). This data therefore suggests that peptides derived from the extracellular C-terminal end of the truncated receptor sst5TMD4 could be used as a method for diagnosis of breast cancer, prostate cancer, liver cancer, and/or neuroendocrine tumors in which sst5TMD4 is present. Furthermore, these extracellular fragments derived from receptor sst5TMD4 (chemically synthesized) increase the malignancy characteristics of cell lines derived from breast cancer, prostate cancer, liver cancer, and neuroendocrine tumors, increasing the capacity of cells to proliferate (Figure 2), migrate (Figure 3), and dedifferentiate (Figure 4). These peptides are also capable of activating certain intracellular signaling routes (such as MEK/ERK and/or PI3K/Akt) (Figure 5) and modulating the expression of genes important in tumor malignancy (MMPs, Ki67, Arp2/3, etc.) (Figure 6). Overall, the obtained data indicates that the processing of the extracellular domain of sst5TMD4 by MMPs could release soluble fragments with pathophysiological effects, such that the blocking therefore may represent a new therapeutic target of pathologies in which the sst5TMD4 is present.

### Description of the Figures

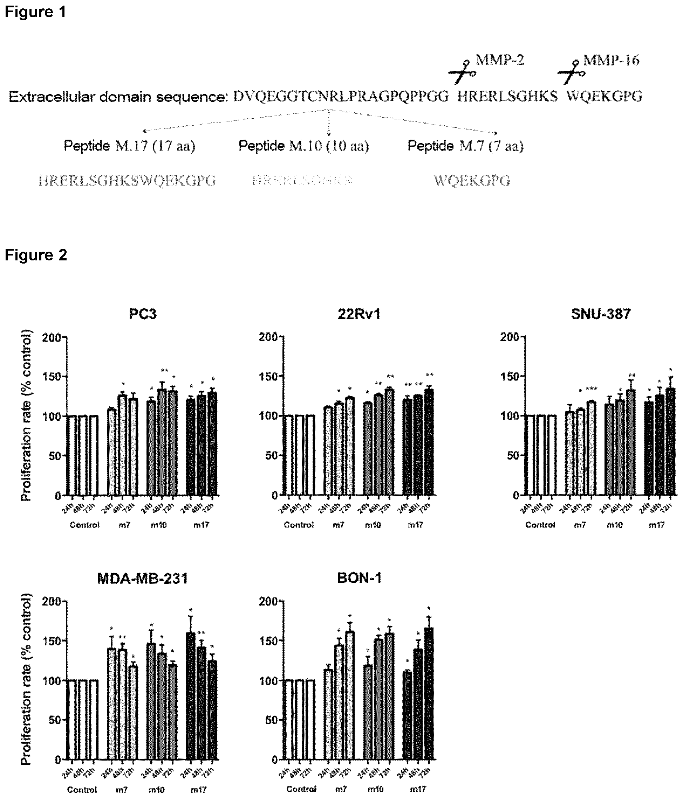

**Figure 1****:** *Schematic representation of the possible metalloprotease cleavage sites in the extracellular domain of receptor sst5TMD4 as well as the sequence of the peptides generated after said cleavage.*
**Figure 2****:** *Effect of treatment with peptides derived from the extracellular end of sst5TMD4 (M7, M10, and M17) on the proliferation of prostate cancer (PC3 and 22Rv1), liver cancer (SNU-387), breast cancer (MDA-MB-231), and neuroendocrine tumor (BON-1) cell lines.* The data represents the means ± SEM. The asterisks (***, p<0.001; **, p<0.01, *, p<0.05) indicate significant differences with respect to the control (vehicle).

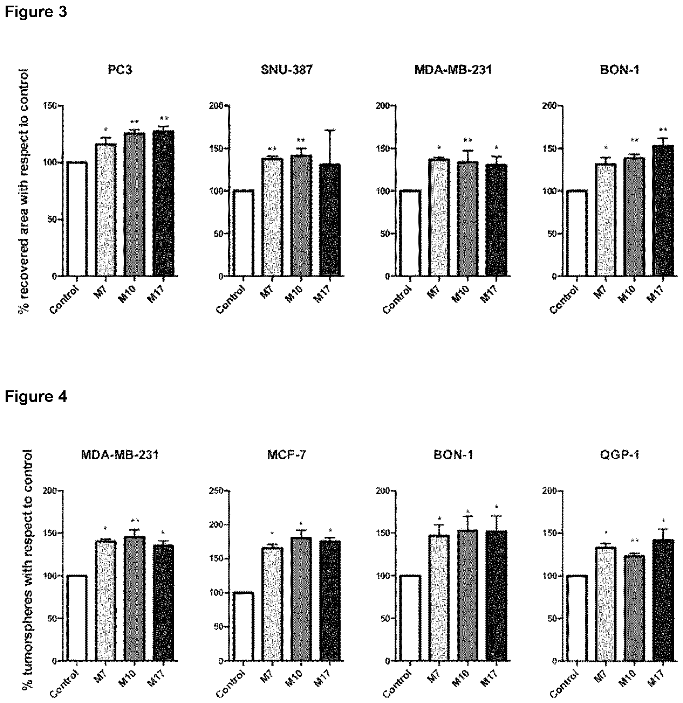

**Figure 3****:** *Effect of treatment with peptides derived from the extracellular end of sst5TMD4 (M7, M10, and M17) on the migration of prostate cancer (PC3), liver cancer (SNU-387), breast cancer (MDA-MB-231), and neuroendocrine tumor (BON-1) cell lines.* The data represent the means ± SEM. The asterisks (***, p<0.001; **, p<0.01, *, p<0.05) indicate significant differences with respect to the control (vehicle).
**Figure 4****:** *Effect of treatment with peptides derived from the extracellular end of sst5TMD4 (M7, M10, and M17) on the tumorsphere formation of breast cancer (MDA-MB-231 and MCF-7) and neuroendocrine tumor (BON-1 and QGP-1) cell lines.* The data represent the means ± SEM. The asterisks (***, p<0.001; **, p<0.01, *, p<0.05) indicate significant differences with respect to the control (vehicle).

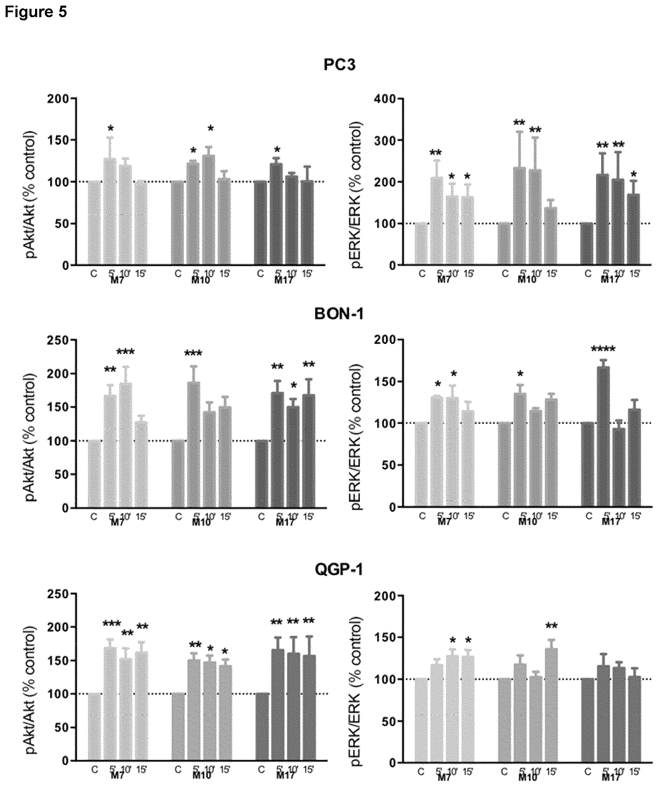

**Figure 5****:** *Effect of treatment with peptides derived from the extracellular end of sst5TMD4 (M7, M10, and M17) on the activation of Akt and ERK signaling routes in prostate cancer (PC3) and neuroendocrine tumor (BON-1 and QGP-1) cell lines.* The data represent the means ± SEM. The asterisks (***, p<0.001; **, p<0.01, *, p<0.05) indicate significant differences with respect to the control (vehicle).

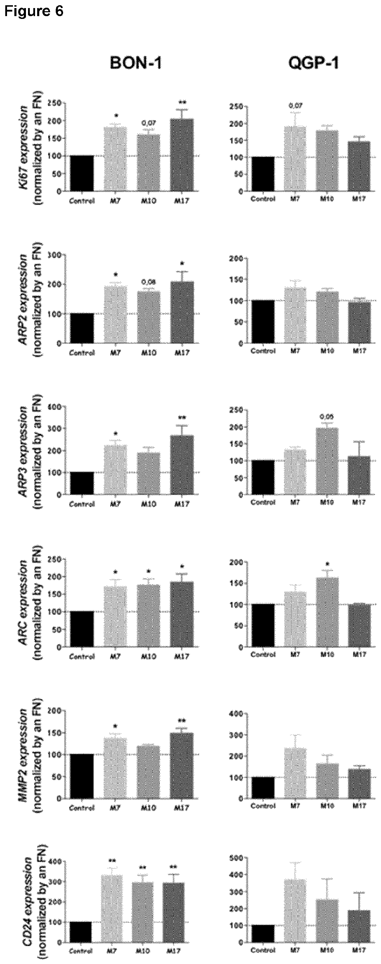

**Figure 6****:** *Effect of treatment with peptides derived from the extracellular end of sst5TMD4 (M7, M10, and M17) on the gene expression of markers relevant in neuroendocrine tumor (BON-1 and QGP-1) cell lines.* The data represent the means ± SEM. The asterisks (***, p<0.001; **, p<0.01, *, p<0.05) indicate significant differences with respect to the control (vehicle).

### Brief Description of the Invention

A growing number of studies suggests that extracellular fragments derived from proteins located in the plasma membrane can play a relevant functional role in the development and/or progression of certain tumors and could therefore serve as new tools for the diagnosis and prognosis of said pathologies. Current interest is focused on one of these proteins, the truncated receptor of somatostatin sst5TMD4, which is expressed in a large number of endocrine tumors, such as breast cancer, prostate cancer, pituitary gland cancer, or neuroendocrine tumors. Unlike other receptors coupled to G proteins, the C-terminal tail of sst5TMD4 presents an extracellular location because it possesses only 4 transmembrane domains, which would allow this domain to be processed by extracellular proteases, and thus release fragments derived from this receptor with a potential biological effect. In fact, an *in silico* approach identified two possible cleavage sites for metalloproteinases MMP-2, -9, -14, and/or MMP-16, the processing of which could generate three soluble fragments with 7, 10, or 17 amino acids. These extracellular fragments derived from receptor sst5TMD4 were chemically synthesized and studied using a battery of functional and molecular assays in cell lines derived from breast cancer, prostate cancer, liver cancer, and neuroendocrine tumors. This demonstrated that the three extracellular fragments of receptor sst5TMD4 increase the characteristics of malignancy of said cell lines, increasing the capacity of cells to proliferate, migrate, and dedifferentiate. Furthermore, these peptides are capable of activating certain intracellular signaling routes (such as MEK/ERK and/or PI3K/Akt) and modulating the expression of important genes in tumor malignancy (MMPs, Ki67, Arp2/3, etc.). Overall, the data obtained indicates that the processing of the extracellular domain of sst5TMD4 by MMPs could release soluble fragments with pathophysiological effects, which suggests that it could be used in diagnosis and/or as a therapeutic target of these pathologies. Therefore, the present invention seeks to claim the use of these peptides as a method for diagnosing and as a therapeutic target in those breast cancers, prostate cancers, liver cancers, and/or neuroendocrine tumors in which the sst5TMD4 may be present.

### Detailed Description of the Invention

In a first aspect, the present invention relates to the use of peptides derived from the extracellular end of truncated receptor sst5TMD4 for the diagnosis, prognosis, and monitoring of cancer. More preferably, peptides derived from the extracellular end of truncated receptor sst5TMD4 are selected from peptides of sequence SEQ ID NO: 1, SEQ ID NO: 2, and SEQ ID NO: 3, or any combination thereof.

In another preferred embodiment, the cancer is selected from breast cancer, prostate cancer, liver cancer, neuroendocrine tumors, or any combination thereof.

### METHODS FOR THE DIAGNOSIS AND PROGNOSIS OF CANCER

A second aspect of the invention relates to a method for obtaining data useful for the diagnosis, prognosis, and monitoring of cancer, hereinafter first method of the invention, comprising:
a) quantifying expression levels of peptides derived from the extracellular end of truncated receptor sst5TMD4 in a biological sample.

In a preferred embodiment of this aspect, the first method of the invention further comprises:
b) comparing the levels obtained in step (a) with a reference value, or with expression levels of peptides derived from the extracellular end of truncated receptor sst5TMD4 obtained in a reference sample;

More preferably, peptides derived from the extracellular end of truncated receptor sst5TMD4 are selected from peptides of sequence SEQ ID NO: 1, SEQ ID NO: 2, and SEQ ID NO: 3, or any combination thereof.

In another preferred embodiment, the cancer is selected from breast cancer, prostate cancer, liver cancer, neuroendocrine tumors, or any combination thereof.

In another preferred embodiment, the biological sample is a sample isolated from plasma, serum, blood, and/or urine of the individual.

Another aspect of the invention relates to a method for the diagnosis, prognosis, and monitoring of cancer, hereinafter second method of the invention, comprising:
a) quantifying expression levels of peptides derived from the extracellular end of truncated receptor sst5TMD4 in a biological sample;
b) comparing the levels obtained in step (a) with a reference value, or with expression levels of peptides derived from the extracellular end of truncated receptor sst5TMD4 obtained in a reference sample; and
c) assigning patients presenting expression levels of peptides derived from the extracellular end of truncated receptor sst5TMD4 obtained in step (b) which are greater than and statistically significant with respect to the reference value or levels of the reference sample, to the group of individuals presenting a risk of suffering cancer or group of individuals who suffer cancer.

Preferably patients presenting expression levels of peptides derived from the extracellular end of truncated receptor sst5TMD4 obtained in step (b) which are greater than and statistically significant with respect to the reference value or levels of the reference sample, are assigned to the group of individuals presenting a risk of suffering cancer.

More preferably, peptides derived from the extracellular end of truncated receptor sst5TMD4 are selected from peptides of sequence SEQ ID NO: 1, SEQ ID NO: 2, and SEQ ID NO: 3, or any combination thereof.

In another preferred embodiment, the cancer is selected from breast cancer, prostate cancer, liver cancer, neuroendocrine tumors, or any combination thereof.

In another preferred embodiment, the biological sample is a sample isolated from plasma, serum, blood, and/or urine of the individual.

The method of quantifying the expression of peptides derived from the extracellular end of truncated receptor sst5TMD4 according to the aspects of the present invention is preferably a method for non-invasive diagnosis of cancer which comprises quantifying the expression of peptides derived from the extracellular end of truncated receptor sst5TMD4 in the plasma, serum, blood, and/or urine of a subject or individual, preferably a subject or individual suspected of suffering cancer and/or at risk of suffering cancer.

Preferably, the *"reference sample"* of the method according to this embodiment of the first aspect of the present invention (method for non-invasive diagnosis) relates to a similar sample obtained from a control subject or individual (patient) who does not present the disease or illness, such as a tumor or cancer, for which a diagnosis is sought.

In a particular embodiment, the method of quantifying the expression of peptides derived from the extracellular end of truncated receptor sst5TMD4 according to the first aspect of the present invention is a method for the prognosis of cancer which comprises the steps of:
a. quantifying expression levels of peptides derived from the extracellular end of truncated receptor sst5TMD4 as defined in SEQ ID NO: 1, 2, or 3, or a sequence with at least 75% identity with SEQ ID NO: 1, 2, or 3 in a plasma, serum, blood, and/or urine sample isolated from an individual suffering cancer;
b. comparing the levels obtained in step (a) with a reference value or with expression levels of peptides derived from the extracellular end of truncated receptor sst5TMD4 as defined in SEQ ID NO: 1, 2, or 3, or a sequence with at least 75% identity with SEQ ID NO: 1, 2, or 3 in a reference sample; and
c. assigning patients presenting expression levels of peptides derived from the extracellular end of truncated receptor sst5TMD4 obtained in step (b) which are greater than and statistically significant with respect to the reference value or expression levels of the reference sample, to the group of individuals with an unfavorable prognosis.

In this embodiment, the *"reference sample"* can be a similar sample obtained from a control subject or individual (patient) who does not present the disease or illness, such as a tumor or cancer, for which a prognosis is sought and/or a similar sample obtained from the same subject or individual at an earlier point in time. When the *"reference sample"* is a similar sample obtained from the same subject or individual at an earlier point in time, the sequence of steps (a)-(b) described above can be performed more than once, such as for example two, three, four, five, six, seven, eight, nine, ten, fifteen, twenty or more times, in plasma, serum, blood, and/or urine samples obtained from one and the same individual, in a non-simultaneous manner. This sequence of steps performed in a non-simultaneous manner provides information useful for the prognosis of disease (cancer) in the patient.

For example, if expression levels of peptides derived from the extracellular end of truncated receptor sst5TMD4 increase with time, it may be indicative that the patient has developed a tumor or that said tumor (which may be pre-existing) has increased in malignancy.

Expression levels of peptides derived from the extracellular end of truncated receptor sst5TMD4 in a plasma sample are quantified based on the levels of protein present in said plasma sample. Therefore, in steps (a) and (b) for quantifying expression levels of peptides derived from the extracellular end of truncated receptor sst5TMD4 in a plasma sample according to the method of the first aspect of the present invention, in any of its variants, the levels of protein as defined in SEQ ID NO: 1, 2, or 3, or a sequence with at least 75% identity, such as 75%, 80%, 85%, 90%, 95%, 97%, or 99%, with SEQ ID NO: 1, 2, or 3, are quantified.

The measurement of the amount or concentration of expression levels, preferably in a semiquantitative or quantitative manner, can be carried out directly or indirectly. Direct measurement refers to the measurement of the amount or concentration of expression products, based on a signal which is obtained directly from expression products and is correlated with the number of molecules of said expression products. Said signal, referred to as intensity signal, can be obtained, for example, by measuring an intensity value of a chemical or physical property of said expression products. Indirect measurement includes the obtained measurement of a secondary component or a biological measurement system (for example the measurement of cellular responses, ligands, *"labels",* or enzymatic reaction products).

Any method known in the state of the art for quantifying the expression of peptides derived from the extracellular end of truncated receptor sst5TMD4 can be used. For example, immunohistochemical means, such as antibodies for example, can be used. These antibodies can be polyclonal or monoclonal antibodies capable of recognizing peptides derived from the extracellular end of truncated receptor sst5TMD4. Antibodies recognizing peptides derived from the extracellular end of truncated receptor sst5TMD4 can be used for carrying out the methods of the present invention by means of an immunoassay. In a preferred embodiment, the detection of peptides derived from the extracellular end of truncated receptor sst5TMD4 can be performed, by way of illustration and without limiting the scope of the present invention, by means of incubating with a specific antibody in an immunoassay. As it is used in the present description, the term *"immunoassay"* refers to any analytical technique which is based on the reaction of the conjugation of an antibody with the obtained sample. Examples of immunoassays known in the state of the art are, for example, but without limitation: immunoblot, enzyme-linked immunosorbent assay (ELISA), radio-immunoanalysis (RIA), immunohistochemistry, or protein microarrays. In another preferred embodiment, the detection of peptides derived from the extracellular end of truncated receptor sst5TMD4 can be performed by means of a radio-immunoanalysis.

A *"plasma, serum, and*/*or blood sample"* refers to a sample obtained by means of a non-invasive method. A non-limiting example known to one skilled in the art would be by means of drawing a blood sample from an individual using a needle. If a plasma or serum sample were to be obtained, the plasma/serum would be separated from the other blood components by means of centrifugation. The amount of peptides derived from the extracellular end of truncated receptor sst5TMD4 can be analyzed, for example, but without limitation, in fresh plasma, serum and/or blood or frozen and then thawed plasma, serum and/or blood.

A *"urine sample"* refers to a sample obtained by means of a non-invasive method. A non-limiting example known to one skilled in the art would be by means of collecting urine from an individual in a sealable container. The amount of peptides derived from the extracellular end of truncated receptor sst5TMD4 can be analyzed, for example, but without limitation, in fresh urine or frozen and then thawed urine samples.

The detection of peptides derived from the extracellular end of truncated receptor sst5TMD4 in a plasma, serum, blood, and/or urine sample at levels which are greater than and/or statistically significant with respect to the reference levels is indicative of said individual suffering cancer, or of said individual presenting the risk of suffering cancer. The terms *"individual", "patient,* or "*subject*" are used interchangeably in the present description and are not intended to be limiting in any aspect, where the "*individual*"*, "patient",* or *"subject"* may be of any age, sex, and physical condition.

Preferably, the cancer or tumor is prostate cancer, breast cancer, liver cancer, and/or neuroendocrine tumors.

Therefore, in the method of the present invention, expression levels of peptides derived from the extracellular end of truncated receptor sst5TMD4 in plasma, serum, blood, and/or urine of a subject can be measured both if the subject has already been diagnosed with the disease (cancer) and if the subject has not (yet) been diagnosed with the disease (cancer).

In a preferred embodiment, the method for the diagnosis and/or prognosis of cancer in a patient also comprises combining the quantification of the protein as defined in SEQ ID NO: 1, 2, or 3, or a sequence with at least 75% identity with SEQ ID NO: 1, 2, or 3, such as 75%, 80%, 85%, 90%, 95%, 97%, or 99% identity with SEQ ID NO: 1 in the plasma, serum, blood, and/or urine with the detection of other biomarker(s), also preferably in plasma, which may improve the precision of said method. For example, but without limitation, the quantification of the expression of peptides derived from the extracellular end of truncated receptor sst5TMD4 could be combined with the detection of PSA (prostate-specific antigen), PCA3 (Prostate CAncer gene 3), GOAT, chromogranin, etc.

### KIT OR DEVICE OF THE INVENTION

Another aspect of the invention relates to a kit or device, hereinafter kit or device of the invention, comprising the elements needed for carrying out any of the methods of the invention.

The kit can further include, without any type of limitation, buffers, protein extraction solutions, agents for preventing contamination, protein degradation inhibitors, etc.

Preferably, the kit or device of the invention comprises at least one antibody capable of binding with SEQ ID NO: 1, SEQ ID NO: 2, SEQ ID NO: 3, or with any combination thereof. It preferably comprises several antibodies capable of binding with said sequences.

In another preferred embodiment of this aspect of the invention, the antibody is human, humanized, or synthetic. In another preferred embodiment, the antibody is monoclonal and/or is labeled with a fluorochrome. Preferably, the fluorochrome is selected from the list comprising fluorescein (FITC), tetramethylrhodamine and derivatives, phycoerythrin (PE), PerCP, Cy5, Texas, allophycocyanin, or any combination thereof.

Another aspect of the invention relates to a protein microarray, hereinafter protein microarray of the invention, comprising antibodies or fragments thereof specific for any of the sequences SEQ ID NO: 1, SEQ ID NO: 2, SEQ ID NO: 3, or any combination thereof, or for nucleotide sequences presenting a degree of identity with said nucleotide sequences of at least 85%, typically at least 90%, preferably at least 95%, more preferably at least 98%, even more preferably at least 99%.

In a preferred embodiment of this aspect of the invention, the protein microarray of the invention has modified antibodies, as described above.

Another aspect of the invention relates to a computer program comprising program instructions for making a computer carry out to practice the method according to any of the methods of the invention.

In particular, the invention covers computer programs arranged on or in a carrier. The carrier can be any entity or device capable of supporting the program. When the program is incorporated in a signal which can be transported directly by a cable or other device or means, the carrier can be made up of said cable or other device or means. As a variant, the carrier could be an integrated circuit in which the program is included, the integrated circuit being adapted for executing, or for being used in the execution of, the corresponding processes.

For example, the programs could be incorporated in a storage medium, such as a ROM memory, a CD ROM memory or a semiconductor ROM memory, a USB memory, or a magnetic recording medium, for example, a floppy disk or a hard disk drive. Alternatively, the programs could be supported in a transmittable carrier signal. For example, it could be an electric or optical signal which could be transported through an electric or optical cable, by radio, or by any other means.

The invention also extends to computer programs adapted so that any processing means can carry out to practice the methods of the invention. Such programs can be in the form of source code, object code, a code intermediate source and object code, for example, in a partially compiled form, or in any other form suitable for use in putting into practice the processes according to the invention. The computer programs also cover applications in the cloud based on said method.

Another aspect of the invention relates to a computer-readable storage medium comprising program instructions capable of making a computer carry out the steps of any of the methods of the invention.

Another aspect of the invention relates to a transmittable signal comprising program instructions capable of making a computer carry out the steps of any of the methods of the invention.

### USE OF PEPTIDES DERIVED FROM THE EXTRACELLULAR END OF TRUNCATED RECEPTOR SST5TMD4 FOR IDENTIFYING CHEMICAL SUBSTANCES USEFUL IN THE TREATMENT OF CANCER

In another aspect, the present invention also could provide a method for using peptides derived from the extracellular end of truncated receptor sst5TMD4 for identifying drugs useful in the treatment of prostate cancer, breast cancer, liver cancer, and/or neuroendocrine tumors.

Therefore, another aspect relates to the use of peptides derived from the extracellular end of truncated receptor sst5TMD4 for identifying drugs useful in the treatment of cancer.

More preferably, peptides derived from the extracellular end of truncated receptor sst5TMD4 are selected from peptides of sequence SEQ ID NO: 1, SEQ ID NO: 2, and SEQ ID NO: 3, or any combination thereof.

In another preferred embodiment, the cancer is selected from breast cancer, prostate cancer, liver cancer, neuroendocrine tumors, or any combination thereof.

Another aspect of the invention relates to a method for identifying drugs useful in the treatment of cancer comprising the steps of:
a. establishing cancer cell lines;
b. quantifying levels of peptides derived from the extracellular end of truncated receptor sst5TMD4 in said cell lines;
c. administering the "test drug" and a control ("vehicle") to the cell lines of (a);
d. quantifying the levels of peptides derived from the extracellular end of truncated receptor sst5TMD4 in said cell lines after the administration of the "test drug" and the "vehicle" according to (c), and/or quantifying any cellular parameter indicative of tumor aggressiveness (cell proliferation, migration, dedifferentiation, etc.) in said cell lines after the administration of the "test drug" and the "vehicle" according to; and
e. assigning the "test drug" according to (c) to the group of chemical substances useful in the treatment of prostate cancer, breast cancer, liver cancer, and/or neuroendocrine tumors when levels of peptides derived from the extracellular end of truncated receptor sst5TMD4 quantified in step (d) are lower than and statistically significant with respect to the levels quantified in step (b) and/or when any of the cellular parameters indicative of tumor aggressiveness (cell proliferation, migration, dedifferentiation, etc.) are significantly lower than those observed in cells treated with the "vehicle".

Preferably the cell lines of step (a) are prostate cancer, breast cancer, liver cancer, and/or neuroendocrine tumor cell lines.

More preferably, peptides derived from the extracellular end of truncated receptor sst5TMD4 are selected from peptides of sequence SEQ ID NO: 1, SEQ ID NO: 2, and SEQ ID NO: 3, or any combination thereof.

In another preferred embodiment, the cancer is selected from breast cancer, prostate cancer, liver cancer, neuroendocrine tumors, or any combination thereof.

Expression levels of peptides derived from the extracellular end of truncated receptor sst5TMD4 in the prostate cancer, breast cancer, liver cancer, and/or neuroendocrine tumor cell lines according to steps (b) and (d) can be quantified based on the levels of protein, as described above.

For example, expression levels of peptides derived from the extracellular end of truncated receptor sst5TMD4 in the prostate cancer, breast cancer, liver cancer, and/or neuroendocrine tumor cell lines according to steps (b) and (d) are quantified based on the levels of protein as defined in SEQ ID NOs.: 1, 2, and/or 3, or of a sequence having at least 75% identity with SEQ ID NOs.: 1, 2, and/or 3, such as 75%, 80%, 85%, 90%, 95%, 97%, or 99% identity with SEQ ID NOs.: 1, 2, and/or 3.

In a preferred embodiment, the first step (a) comprises establishing prostate cancer, breast cancer, liver cancer, and/or neuroendocrine tumor cell lines. The cancer cell lines can be established by means of any method known to one skilled in the art. In another preferred embodiment, cancer cell lines already established, such as, for example, but without limitation, cancer cell lines PC3, DU145, LNCaP, VCaP, MCF7, MDA-MB-231, BON-1, QGP-1, HEPG2, HEP3B, and/or SNU387, are used.

The term *"test drug"* refers to a material with a defined chemical composition made up of its entities: molecules, formula units, and atoms. In a preferred embodiment, the test drugs present anticancer properties. In another preferred embodiment, the test drugs with anticancer properties are identified in a sample library of compounds by means of the systematic identification of compounds.

The systematic identification of compounds can be carried out by means of any method known to one skilled in the art, for example, but without limitation, predictions *in silico* of inhibitory molecules or antagonists, high-throughput assays (HTAs), etc.

### Definitions

Unless specifically otherwise mentioned, the term *"comprises"* is used, in the context of this application, to indicate that the list of components can include optional aspects that may or may not be mentioned. The term *"comprises"* can also include the concept *"consists of'.*

A "*diagnostic method*" (or *"method for diagnosis*") refers to a method where a patient (or a sample obtained therefrom) is examined or analyzed so as to obtain differential knowledge of a disease through the signs and symptoms which characterize them. In summary, the term *"diagnosis"* in the present application refers to the capacity to discriminate between patients who suffer cancer and patients who do not suffer cancer. As it is understood by one skilled in the art, this discrimination does not intend to be correct in 100% of the analyzed samples. However, it requires that a statistically significant amount of the analyzed samples are correctly classified. The amount that is statistically significant can be established by one skilled in the art by means of using different statistical tools, for example, but without limitation, by means of the determination of confidence intervals, determination of the p-value, Student's t-test or Fisher discriminant functions. Preferably, the confidence intervals are at least 90%, at least 95%, at least 97%, at least 98%, or at least 99%. Preferably, the p-value is less than 0.1, 0.05, 0.01, 0.005, or 0.0001. Preferably, the present invention allows correctly detecting the disease in a differential manner in at least 60%, in at least 70%, in at least 80%, or in at least 90% of the subjects of a given analyzed group or population.

The degree of identity existing between two sequences can be determined by conventional methods, for example, by means of standard sequence alignment algorithms which are known in the state of the art, such as for example BLAST (Altschul S.F. et al. Basic local alignment search tool. J Mol Biol. 1990 Oct 5; 215(3):403-10).

The peptides derived from truncated somatostatin receptor sst5TMD4 refer to a protein the amino acid sequence of which comprises or, alternatively, consists of, SEQ ID NO: 1, 2, or 3, or presents an identity of at least 75% identity with SEQ ID NO: 1, 2, or 3, such as 80, 85, 90, 95, 97, or 99% identity with SEQ ID NO: 1, 2, or 3, or a fragment thereof. For example, the peptides derived from truncated somatostatin receptor sst5TMD4 can refer to a fragment of peptides derived from truncated somatostatin receptor sst5TMD4 comprising the 5 amino acids of the *N*-terminal end of SEQ ID NO: 1, 2, or 3, or presents an identity of at least 75% identity with SEQ ID NO: 1, 2, or 3, such as 80, 85, 90, 95, 97 or 99% identity with SEQ ID NO: 1, 2, or 3.
SEQ ID NO: 1
   HRERLSGHKSWQEKGPG (Peptide M.17)
SEQ ID NO: 2
   HRERLSGHKS (Peptide M.10)
SEQ ID NO: 3
   WQEKGPG (Peptide M.7).

A *"reference sample"* refers to a similar sample obtained from a control subject or individual (patient) who does not present the disease or illness, such as a tumor or cancer, for which a diagnosis or prognosis is sought. The *"reference amount'* or *"reference level*" refers to the absolute amount (or levels) obtained from the reference sample. A *"reference sample"* can also refer to a similar sample obtained from the same subject or individual at an earlier point in time.

A *"reference value"* refers to the set of values which are used to interpret the results of the diagnostic and/or prognostic tests in a subject or individual (patient). For example, the reference values for a given test are based on the results of that same test in 95% of the healthy population. The reference values of a test can be different in different groups of people (for example, between women and men). It can also be referred to as *"reference interval*"*, "reference limit*"*,* and *"normal limit*".

As it is used in the present description, the term *"amount'* refers but is not limited to the absolute or relative amount of the expression products (mRNA and/or protein), as well as any other value or parameter related thereto or which can be derived from the same. Said values or parameters comprise signal intensity values obtained based on any of the physical or chemical properties of the expression products obtained by means of direct measurement. Additionally, said values or parameters include all those obtained by means of indirect measurement, for example, any of the measurement systems described in another part of this document.

As it is used in the present description, the term "*antibody*" refers to molecules of immunoglobulins and immunologically active fragments of immunoglobulin molecules, i.e., molecules containing an antigen fixing site which specifically binds (is immunoreactive) with, for example, any of peptides derived from the extracellular end of truncated receptor sst5TMD4. Examples of immunologically active fragments include fragments F(ab) and F(ab')₂ which can be generated by treating the antibody with an enzyme such as pepsin. The antibodies can be polyclonal (typically including different antibodies targeting different determinants or epitopes) or monoclonal (targeting a single determinant in the antigen).

The monoclonal antibody can be biochemically altered by genetic manipulation, or it can be synthetic, possibly lacking the antibody in its entirety or partially, of fragments or portions which are not needed for recognizing the antigen, such as for example peptides derived from the extracellular end of truncated receptor sst5TMD4, and being substituted with others conferring additional advantageous properties to the antibody.

The antibody can be also recombinant, chimeric, humanized, synthetic, or a combination of any of the foregoing. A *"recombinant antibody or polypeptide"* (rAC) is an antibody which has been produced in a host cell which has been transformed or transfected with the nucleic acid encoding the polypeptide or antigen, or producing the polypeptide or antigen as a result of homologous recombination.

As it is used in the present description, the term *"non-simultaneous"* refers to the acquisition of two or more tissue, blood (which also includes plasma and serum), or urine samples where there is a time interval between the acquisition of the samples which allows the monitoring of cancer and/or useful prognosis by means of the comparison between the amounts of mRNA and/or proteins in the different samples. Preferably, the time interval is between 1 month and 20 years, such as for example between 1 month and 10 years, 1 month and 5 years or 1 month and 1 year. For example, the time interval between the acquisition of samples is about 1 month. For example, the time interval between the acquisition of samples is about 2 months. For example, the time interval between the acquisition of samples is about 3 months. For example, the time interval between the acquisition of samples is about 6 months. For example, the time interval between the acquisition of samples is about 1 year. For example, the time interval between the acquisition of samples is about 1 year and a half. For example, the time interval between the acquisition of samples is about 2 years. For example, the time interval between the acquisition of samples is about 3 years. For example, the time interval between the acquisition of samples is about 5 years.

The terms "amino acid sequence", "peptide", "oligopeptide", "polypeptide" and "protein" are used herein interchangeably and refer to a polymeric form of amino acids of any length, which can be encoding or non-encoding, chemically or biochemically modified.

Throughout the description and claims, the word "comprises" and its variants do not intend to exclude other technical features, additives, components, or steps. For those skilled in the art, other objects, advantages, and features of the invention are inferred in part from the description and in part from the practice of the invention. The following examples and drawings are provided by way of illustration and do not intend to be limiting of the present invention.

### Examples

### Example 1: Determination in silico of the cleavage sites for metalloproteases in the extracellular domain of sst5TMD4 and prediction of the peptides derived from the processing thereof.

The presence of possible cleavage sites for proteases in the extracellular segment of sst5TMD4 was analyzed using the CleavPredict software, and two cleavage sites which could bring about the generation of 3 possible peptides resulting from proteolysis by means of metalloproteases MMP-2,9,14,16 were found. These peptides would have a length of 7, 10, and 17 amino acids (M7, M10, and M17, respectively) since they would be cleaved at positions 200 (MMP-2,9,14) and 210 (MMP-16). They were chemically synthesized (Genosphere Biotech).

### Example 2: Effect of peptides derived from the extracellular domain of sst5TMD4 on the proliferative capacity of cell lines.

Cell lines derived from breast cancer (MDA-MB-231 and MCF-7), prostate cancer (PC-3 and 22Rv1), liver cancer (SNU-387), and neuroendocrine tumors (BON-1 and QGP-1) were obtained from the ATCC and kept in culture using standard conditions recommended by the supplier. Cell proliferation/viability was determined by means of an Alamar Blue reagent or MTT tetrazolium salt colorimetric assay. For the Alamar Blue assays, cells were seeded at a density of 3,000-5,000 in 96-well plates and deprived of serum for 24 hours. The cells were treated with serum-free medium and 10% Alamar Blue for 3-4 hours. The reduction in levels of Alamar Blue was analyzed by means of excitation at 560 nm and detection at 590 nm using the FlexStation III system. For MTT assays, cells were seeded at a density of 10,000 cells/well in 96-well plates. After 36 hours of culture, the culture medium was removed and fresh culture medium containing 1% FBS was added. Before measuring, cells were incubated with a solution containing MTT for 2 hours, the solution was removed, and a solution with DMSO, SDS, and acetic acid was added and the cells were centrifuged at 150 rpm for 15 minutes. The measurements were taken with a FlexStation III at 590 nm. In all cases, after a baseline determination, the cultures were treated with a 10⁻⁷ M concentration of each peptide and the proliferation was determined after 24, 48, and 72 hours of incubation.

These studies demonstrated that the treatment with peptides derived from the extracellular end of sst5TMD4 can increase the proliferation of all the treated tumor cell lines (PC-3, 22Rv1, SNU-387, MDA-MB-231, and BON-1) after 24, 48, and/or 72 hours (Figure 2).

### Example 3: Effect of peptides derived from the extracellular domain of sst5TMD4 on the migration capacity of cell lines.

Cell migration was determined by means of a wound healing assay. 200,000 cells were seeded in 6-well culture plates and cultured until confluence. A streak (wound) was then made on the surface with a 200 µl pipette tip and the cells were incubated at 37°C for 12-24 hours (depending on the cell line). Three independent photographs were taken at 0 hours and 12 hours after the wound. Wound repair was calculated as the area of the rectangle centered in the photograph which was taken 12 hours after the wound against the area of the rectangle right after having streaked the surface.

These studies demonstrated that the treatment with peptides derived from the extracellular end of sst5TMD4 can increase the migration of all the treated tumor cell lines (PC-3, SNU-387, MDA-MB-231, and BON-1) (Figure 3).

### Example 4: Effect of peptides derived from the extracellular domain of sst5TMD4 on the dedifferentiation capacity (tumorsphere formation) of cell lines.

For the tumorsphere formation studies, 2,000-6,000 cells per well (depending on the cell line) were seeded in 6-well plates with a very low adherence capacity (for which they were treated with 1 ml of p-HEMA and kept in an oven at 40°C for 3 days). At the time of seeding, the medium was supplemented with the peptides of interest and refreshed every two days. The cells were kept under these conditions for one week, and the number of tumorspheres were then counted under a microscope.

These studies demonstrated that the treatment with peptides derived from the extracellular end of sst5TMD4 can increase the capacity of forming tumorspheres of all the treated tumor cell lines (MDA-MB-231, MCF-7, BON-1, and QGP-1) (Figure 4).

### Example 5: Effect of peptides derived from the extracellular domain of sst5TMD4 on the signaling route activation of cell lines.

In order to explain by means of which signaling routes act peptides derived from the extracellular region of sst5TMD4, Western blot studies were carried out. Namely, the phosphorylation levels of Akt and ERK were measured. To that end, the cells were lysed with a lysis buffer (62.5 mM Tris-HCI, 2% SDS, 20% glycerol, 100 mM DTT, and 0.005% bromophenol blue) and sonicated 3 times for 5 seconds. Next, they were heated for 5 minutes at 95°C and then centrifuged at 13,000 rpm for 5 minutes. The samples were loaded in 12.5% acrylamide gel and run for an hour and a half, first at 80 V and then at 120 V. Next, the proteins were transferred to a nitrocellulose membrane at 90 V for 60 minutes. Then 3 10-minute washes were performed using TTBS (saline buffer with 0.1% Tween). To prevent the non-specific binding of the antibody, the membrane was then treated with TTBSL (TTBS with 5% powder milk) for 1 hour. Next, the corresponding primary antibodies dissolved in TTBSL at a concentration of 1:1000 were added. The membranes with the antibodies were incubated overnight at 4°C. Next, 3 washes with TTBS were performed again. Then a dilution was prepared with the secondary antibody (1:1000) and the membranes were incubated for 1 hour in the presence of this antibody. Three 10-minute washes with TTBS were performed again, and finally the membranes were developed using the Pierce ECL Selectet 2 Western Blotting Detection Reagent RPN 2235 kit (GE Healthcare). Levels of total Akt, phosphorylated Akt, total ERK, and phosphorylated ERK were determined and analyzed by means of Image J software. Levels of total Akt and ERK were used as a control to normalize phosphorylation levels.

These studies demonstrated that the treatment with peptides derived from the extracellular end of sst5TMD4 increases activation of the ERK and Akt signaling routes in the treated tumor cell lines (PC-3, BON-1, and QGP-1) (Figure 5).

### Example 6: Effect of peptides derived from the extracellular domain of sst5TMD4 on the gene expression of cell lines.

In order to determine the effect of peptides derived from the extracellular domain of sst5TMD4 on the gene expression of cell lines, 200,000 cells were seeded in 6-well culture plates and cultured until sub-confluence. Then the cells were treated with the peptides (and the vehicle) and collected after 24h in Trizol, which allows extracting the mRNA following the manufacturer's instructions. 1000 ng of RNA were retrotranscribed to cDNA using a set of random primers following the manufacturer's indications (RevertAid First Strand cDNA, Thermo Scientific). The cDNA obtained from retrotranscription was amplified by qPCR using the thermal cycler Mx3000P (Stratagene, Madrid, Spain) and Brilliant III SYBR Green QPCR Master Mix (Agilent, Madrid, Spain). This technique allowed knowing the absolute values (number of copies) of mRNA of the genes of interest in the analyzed samples.

These studies demonstrated that the treatment with peptides derived from the extracellular end of sst5TMD4 increases the expression of key tumor markers (Ki67, Arp2/3 complex, MMP2, or CD24) in the treated tumor cell lines (BON-1 and QGP-1) (Figure 6).

### References:

Balduyck, M., F. Zerimech, V. Gouyer, R. Lemaire, B. Hemon, G. Grard, C. Thiebaut, V. Lemaire, E. Dacquembronne, T. Duhem, A. Lebrun, M. J. Dejonghe and G. Huet (2000). "Specific expression of matrix metalloproteinases 1, 3, 9 and 13 associated with invasiveness of breast cancer cells in vitro." Clin Exp Metastasis 18(2): 171-178.
Brinckerhoff, C. E. and L. M. Matrisian (2002). "Matrix metalloproteinases: a tail of a frog that became a prince." Nat Rev Mol Cell Biol 3(3): 207-214.
Butler, G. S. and C. M. Overall (2009). "Proteomic identification of multitasking proteins in unexpected locations complicates drug targeting." Nat Rev Drug Discov 8(12): 935-948.
Butler, G. S. and C. M. Overall (2009). "Updated biological roles for matrix metalloproteinases and new "intracellular" substrates revealed by degradomics." Biochemistry 48(46): 10830-10845. Chen, J. and L. U. Thompson (2003). "Lignans and tamoxifen, alone or in combination, reduce human breast cancer cell adhesion, invasion and migration in vitro." Breast Cancer Res Treat 80(2): 163-170.
Cheng, C. W., J. C. Yu, H. W. Wang, C. S. Huang, J. C. Shieh, Y. P. Fu, C. W. Chang, P. E. Wu and C. Y. Shen (2010). "The clinical implications of MMP-11 and CK-20 expression in human breast cancer." Clin Chim Acta 411(3-4): 234-241.
Csaba, Z., S. Peineau and P. Dournaud (2012). "Molecular mechanisms of somatostatin receptor trafficking." J Mol Endocrinol 48(1): R1-12.
Davies, K. J. (2014). "The Complex Interaction of Matrix Metalloproteinases in the Migration of Cancer Cells through Breast Tissue Stroma." Int J Breast Cancer 2014: 839094.
Duran-Prado, M., M. D. Gahete, M. Hergueta-Redondo, A. J. Martinez-Fuentes, J. Cordoba-Chacon, J. Palacios, F. Gracia-Navarro, G. Moreno-Bueno, M. M. Malagon, R. M. Luque and J. P. Castano (2012). "The new truncated somatostatin receptor variant sst5TMD4 is associated to poor prognosis in breast cancer and increases malignancy in MCF-7 cells." Oncogene 31(16): 2049-2061.
Duran-Prado, M., A. Saveanu, R. M. Luque, M. D. Gahete, F. Gracia-Navarro, P. Jaquet, H. Dufour, M. M. Malagon, M. D. Culler, A. Barlier and J. P. Castano (2010). "A potential inhibitory role for the new truncated variant of somatostatin receptor 5, sst5TMD4, in pituitary adenomas poorly responsive to somatostatin analogs." J Clin Endocrinol Metab 95(5): 2497-2502. Hadler-Olsen, E., B. Fadnes, I. Sylte, L. Uhlin-Hansen and J. O. Winberg (2011). "Regulation of matrix metalloproteinase activity in health and disease." FEBS J 278(1): 28-45.
Hadler-Olsen, E., J. O. Winberg and L. Uhlin-Hansen (2013). "Matrix metalloproteinases in cancer: their value as diagnostic and prognostic markers and therapeutic targets." Tumour Biol 34(4): 2041-2051.
Havt, A., A. V. Schally, G. Halmos, J. L. Varga, G. L. Toller, J. E. Horvath, K. Szepeshazi, F. Koster, K. Kovitz, K. Groot, M. Zarandi and C. A. Kanashiro (2005). "The expression of the pituitary growth hormone-releasing hormone receptor and its splice variants in normal and neoplastic human tissues." Proc Natl Acad Sci U S A 102(48): 17424-17429.
Hawrylyshyn, K. A., G. A. Michelotti, F. Coge, S. P. Guenin and D. A. Schwinn (2004). "Update on human alpha1-adrenoceptor subtype signaling and genomic organization." Trends Pharmacol Sci 25(9): 449-455.
Jiang, W. G., G. Davies, T. A. Martin, C. Parr, G. Watkins, M. D. Mason and R. E. Mansel (2006). "Expression of membrane type-1 matrix metalloproteinase, MT1-MMP in human breast cancer and its impact on invasiveness of breast cancer cells." Int J Mol Med 17(4): 583-590.
Kilpatrick, G. J., F. M. Dautzenberg, G. R. Martin and R. M. Eglen (1999). "7TM receptors: the splicing on the cake." Trends Pharmacol Sci 20(7): 294-301.
Klagge, A., K. Krause, K. Schierle, F. Steinert, H. Dralle and D. Fuhrer (2010). "Somatostatin receptor subtype expression in human thyroid tumours." Horm Metab Res 42(4): 237-240. Kohrmann, A., U. Kammerer, M. Kapp, J. Dietl and J. Anacker (2009). "Expression of matrix metalloproteinases (MMPs) in primary human breast cancer and breast cancer cell lines: New findings and review of the literature." BMC Cancer 9: 188.
Luque, R. M., A. Ibanez-Costa, L. V. Neto, G. F. Taboada, D. Hormaechea-Agulla, L. Kasuki, E. Venegas-Moreno, A. Moreno-Carazo, M. A. Galvez, A. Soto-Moreno, R. D. Kineman, M. D. Culler, M. D. Gahete, M. R. Gadelha and J. P. Castano (2015). "Truncated somatostatin receptor variant sst5TMD4 confers aggressive features (proliferation, invasion and reduced octreotide response) to somatotropinomas." Cancer Lett 359(2): 299-306.
Magee, P. J., H. McGlynn and I. R. Rowland (2004). "Differential effects of isoflavones and lignans on invasiveness of MDA-MB-231 breast cancer cells in vitro." Cancer Lett 208(1): 35-41. McWilliams, D. F., S. A. Watson, D. M. Crosbee, D. Michaeli and R. Seth (1998). "Coexpression of gastrin and gastrin receptors (CCK-B and delta CCK-B) in gastrointestinal tumour cell lines." Gut 42(6): 795-798.
Nagase, H., R. Visse and G. Murphy (2006). "Structure and function of matrix metalloproteinases and TIMPs." Cardiovasc Res 69(3): 562-573.
Patel, Y. C. (1999). "Somatostatin and its receptor family." Front Neuroendocrinol 20(3): 157-198. Pisarek, H., T. Stepien, R. Kubiak, E. Borkowska and M. Pawlikowski (2009). "Expression of somatostatin receptor subtypes in human thyroid tumors: the immunohistochemical and molecular biology (RT-PCR) investigation." Thyroid Res 2(1): 1.
Puig-Domingo, M., R. M. Luque, J. L. Reverter, L. M. Lopez-Sanchez, M. D. Gahete, M. D. Culler, G. Diaz-Soto, F. Lomena, M. Squarcia, J. L. Mate, M. Mora, L. Fernandez-Cruz, O. Vidal, A. Alastrue, J. Balibrea, I. Halperin, D. Mauricio and J. P. Castano (2014). "The Truncated Isoform of Somatostatin Receptor5 (sst5TMD4) Is Associated with Poorly Differentiated Thyroid Cancer." PLoS One 9(1): e85527.
Rekasi, Z., T. Czompoly, A. V. Schally and G. Halmos (2000). "Isolation and sequencing of cDNAs for splice variants of growth hormone-releasing hormone receptors from human cancers." Proc Natl Acad Sci U S A 97(19): 10561-10566.
Sampedro-Nunez, M., R. M. Luque, A. M. Ramos-Levi, M. D. Gahete, A. Serrano-Somavilla, A. Villa-Osaba, M. Adrados, A. Ibanez-Costa, E. Martin-Perez, M. D. Culler, M. Marazuela and J. P. Castano (2015). "Presence of sst5TMD4, a truncated splice variant of the somatostatin receptor subtype 5, is associated to features of increased aggressiveness in pancreatic neuroendocrine tumors." Oncotarget 7(6): 6593-6608.
Siegel, R. L., K. D. Miller and A. Jemal (2017). "Cancer Statistics, 2017." CA Cancer J Clin 67(1): 7-30.
Somiari, S. B., R. I. Somiari, C. M. Heckman, C. H. Olsen, R. M. Jordan, S. J. Russell and C. D. Shriver (2006). "Circulating MMP2 and MMP9 in breast cancer -- potential role in classification of patients into low risk, high risk, benign disease and breast cancer categories." Int J Cancer 119(6): 1403-1411.
Stankovic, S., G. Konjevic, K. Gopcevic, V. Jovic, M. Inic and V. Jurisic (2010). "Activity of MMP-2 and MMP-9 in sera of breast cancer patients." Pathol Res Pract 206(4): 241-247.
Unger, N., B. Ueberberg, S. Schulz, W. Saeger, K. Mann and S. Petersenn (2012). "Differential expression of somatostatin receptor subtype 1-5 proteins in numerous human normal tissues." Exp Clin Endocrinol Diabetes 120(8): 482-489.
Viollet, C., G. Lepousez, C. Loudes, C. Videau, A. Simon and J. Epelbaum (2008). "Somatostatinergic systems in brain: networks and functions." Mol Cell Endocrinol 286(1-2): 75-87.
Watt, H. L., G. Kharmate and U. Kumar (2008). "Biology of somatostatin in breast cancer." Mol Cell Endocrinol 286(1-2): 251-261.
Zhao, Y. G., A. Z. Xiao, J. Ni, Y. G. Man and Q. X. Sang (2009). "Expression of matrix metalloproteinase-26 in multiple human cancer tissues and smooth muscle cells." Ai Zheng 28(11): 1168-1175.

## Claims

1. A method for obtaining data useful for the diagnosis, prognosis, and monitoring of cancer comprising:
a) quantifying levels of peptides derived from the extracellular end of truncated receptor sst5TMD4 in a biological sample.

2. The method according to claim 1 further comprising:
b) comparing the levels obtained in step (a) with a reference value, or with expression levels of peptides derived from the extracellular end of truncated receptor sst5TMD4 obtained in a reference sample.

3. A method for the diagnosis, prognosis, and monitoring of cancer comprising steps (a) and (b) according to any of claims 1-2, and further comprising:
c) assigning patients presenting expression levels of peptides derived from the extracellular end of truncated receptor sst5TMD4 obtained in step (b) which are greater than and statistically significant with respect to the reference value or levels of the reference sample, to the group of individuals presenting a risk of suffering cancer or group of individuals who suffer cancer.

4. The method according to any of claims 1-3, wherein peptides derived from the extracellular end of truncated receptor sst5TMD4 are selected from peptides of sequence SEQ ID NO: 1, SEQ ID NO: 2, and SEQ ID NO: 3, or a sequence with at least 75% identity with sequences SEQ ID NO: 1, SEQ ID NO: 2, and SEQ ID NO: 3, respectively, or any combination thereof.

5. The method according to any of claims 1-4, wherein the cancer is selected from breast cancer, prostate cancer, liver cancer, neuroendocrine tumors, or any combination thereof.

6. The method according to any of claims 1-6, wherein the biological sample is a sample isolated from plasma, serum, blood, and/or urine of the individual.

7. The method according to any of claims 1-7, quantifying levels of peptides derived from the extracellular end of truncated receptor sst5TMD4 is performed by an immunohistochemical method.

8. The method according to any of claims 1-7, quantifying levels of peptides derived from the extracellular end of truncated receptor sst5TMD4 is performed by means of an immunoassay.

9. The method according to claim 9, wherein the immunoassay is an immunoblot, enzyme-linked immunosorbent assay (ELISA), radio-immunoanalysis (RIA), immunohistochemistry, or protein microarrays.

10. A kit or device for the diagnosis, prognosis, and monitoring of cancer which comprises the elements needed for carrying out any of the methods of the invention comprising at least one antibody capable of binding with SEQ ID NO: 1, SEQ ID NO: 2, SEQ ID NO: 3, or any combination thereof.

11. Use of peptides derived from the extracellular end of truncated receptor sst5TMD4 for identifying drugs useful in the treatment of cancer.

12. The use of the peptides according to the preceding claim, wherein peptides derived from the extracellular end of truncated receptor sst5TMD4 are selected from peptides of sequence SEQ ID NO: 1, SEQ ID NO: 2, and SEQ ID NO: 3, or a sequence with at least 75% identity with sequences SEQ ID NO: 1, SEQ ID NO: 2, and SEQ ID NO: 3, respectively, or any combination thereof.

13. The use of the peptides according to any of claims 12-13, wherein the cancer is selected from breast cancer, prostate cancer, liver cancer, neuroendocrine tumors, or any combination thereof.

14. A method for identifying drugs useful in the treatment of cancer comprising the steps of:
(a) establishing cancer cell lines;
(b) quantifying levels of peptides derived from the extracellular end of truncated receptor sst5TMD4 in said cell lines;
(c) administering the "test drug" and a control ("vehicle") to the cell lines of (a);
(d) quantifying the levels of peptides derived from the extracellular end of truncated receptor sst5TMD4 in said cell lines after the administration of the "test drug" and the "vehicle" according to (c), and/or quantifying any cellular parameter indicative of tumor aggressiveness (cell proliferation, migration, dedifferentiation, etc.) in said cell lines after the administration of the "test drug" and the "vehicle" according to; and
(e) assigning the "test drug" according to (c) to the group of chemical substances useful in the treatment of prostate cancer, breast cancer, liver cancer, and/or neuroendocrine tumors when levels of peptides derived from the extracellular end of truncated receptor sst5TMD4 quantified in step (d) are lower than and statistically significant with respect to the levels quantified in step (b), and/or when any of the cellular parameters indicative of tumor aggressiveness (cell proliferation, migration, dedifferentiation, etc.) are significantly lower than those observed in cells treated with the "vehicle".

15. The method according to the preceding claim, wherein peptides derived from the extracellular end of truncated receptor sst5TMD4 are selected from peptides of sequence SEQ ID NO: 1, SEQ ID NO: 2, and SEQ ID NO: 3, or a sequence with at least 75% identity with sequences SEQ ID NO: 1, SEQ ID NO: 2, and SEQ ID NO: 3, respectively, or any combination thereof.

16. The method according to any of claims 15-16, wherein the cancer is selected from breast cancer, prostate cancer, liver cancer, neuroendocrine tumors, or any combination thereof.
